# EUROPEAN PATENT APPLICATION

(11) **EP 4 813 516 A1**
(43) Date of publication of application: **30.09.2026**
(21) Application number: 25166961.0
(22) Date of filing: 28.03.2025
(51) Int. Cl.: B01L 3/00, C12M 1/00

(54) **DEVICE AND METHOD FOR EXTRACTING AND DETECTING BACTERIA**

(71) Applicant: Technische Universität München, in Vertretung des Freistaates Bayern, 80333 München (DE)
(72) Inventor: Sabersky-Müssigbrodt, Henning, 80469 München (DE); Hayden, Oliver, 85368 Moosburg (DE); Wali, Sarah, 81373 München (DE)
(74) Representative: Lucke, Andreas

(57) **Abstract**

A device (2) for detecting bacteria (36) contained in a fluid is provided. The device (2) comprises an inlet (4) arranged on the device (2). The inlet (4) is adapted to permit the fluid to enter the device (2) therethrough. The device (2) comprises a filter (6) adapted to retain the bacteria contained in the fluid, and a bacterial-sensitive indicator (8) adapted to indicate a presence of the bacteria (36). The bacterial-sensitive indicator (8) is arranged at a position horizontally offset from a position of the inlet (4). The filter (6) is arranged moveably between a first position below the inlet and a second position below the bacterial-sensitive indicator (8).

## Description

### FIELD OF THE DISCLOSURE

The present disclosure relates to a device and a method for extracting and detecting bacteria contained in fluids, in particular in urine. Specifically, the present disclosure pertains to a device and a method implementing backwashing techniques to extract bacteria from urine. For this purpose, the device comprises a movable filter that retains bacteria. An inlet of the device and a bacterial-sensitive indicator are both positioned above the movable filter, according to different positions of the movement, thus implementing the specific device for the backwashing. The method implements a backwashing technique to extract bacteria from urine.

### BACKGROUND

Devices and methods for analyzing urine to detect urinary tract infections typically involve two main techniques: urine cultures and urine dipstick tests.

A urine culture involves collecting a urine sample and placing it in a culture medium that promotes bacterial growth. The sample is then incubated for a specified period, usually 24-48 hours, allowing any bacteria present to multiply. The culture medium is often contained within a petri dish or a similar container, and the growth of bacteria is monitored visually or with the aid of a microscope. The presence of bacterial colonies indicates an infection, and further tests can identify the specific type of bacteria and its antibiotic susceptibility.

Urine dipstick tests work easier and faster. They involve dipping a specially treated strip into a urine sample. The strip contains various chemical reagents that react with substances in the urine, such as nitrites, leukocyte esterase, and proteins, which are indicative of bacterial infection. The strip changes color based on the presence and concentration of these substances, and the results are compared against a color chart to interpret the findings.

Economically, these devices and methods are crucial in healthcare for the early detection and treatment of urinary tract infections, reducing the risk of complications and the spread of infections. Technologically, advancements in these devices have led to more rapid, accurate, and user-friendly diagnostic tools, improving patient outcomes and streamlining clinical workflows. Scientifically, these methods contribute to a better understanding of bacterial behavior and resistance patterns, aiding in the development of new antibiotics and treatment protocols.

### SUMMARY OF THE DISCLOSURE

It is an object of this invention to provide improvements in relation to the detection of bacteria from urine, and, in particular, to provide an improved device for detecting bacteria contained in a fluid such as urine, as well as an improved method for extracting bacteria contained in urine.

According to an aspect of the disclosure, a device for detecting bacteria contained in a fluid is provided. The device comprises an inlet arranged on the device. The inlet is adapted to permit the fluid to enter the device therethrough. The device comprises a filter adapted to retain the bacteria contained in the fluid, and a bacterial-sensitive indicator adapted to indicate a presence of the bacteria. The bacterial-sensitive indicator is arranged at a position horizontally offset from a position of the inlet. The filter is arranged moveably between a first position below the inlet and a second position below the bacterial-sensitive indicator.

Both the inlet and the bacterial-sensitive indicator are arranged on the same side, namely above, the filter, according to the first and second position. Thus, the device is optimized for detecting the bacteria using a backwashing technique. The device is designed for the fluid potentially containing the bacteria, such as urine, to be flown onto the filter from above when the filter is at the first position. The bacteria are thus retained on the filter on its upper surface. The filter can then be moved to the second position. The device is designed such that, when the backwashing is performed with the filter at the second position, the backwashing brings the bacteria to the bacterial-sensitive indicator arranged above the second position.

The device is thus optimized for providing the bacteria to the bacterial-sensitive indicator in a purified form, i.e. filtered from undersized (with respect to the bacteria, or with respect to a pore size of the filter, respectively) contaminants.

The inventors have realized, that using this approach, fast and economic analysis methods for the analysis of the urine and for detecting urinary tract infections are made possible or are significantly improved.

This applies, in particular, to paper-based diagnostic methods. In the context of this disclosure, paper-based diagnostic methods may refer to methods wherein the bacterial-sensitive indicator is provided in the form of a paper, such as a paper strip or a blotting paper or a blotting paper strip, impregnated with the bacterial-sensitive indicator. Alternatively, paper-based diagnostic methods, in the context of this disclosure, may refer to methods wherein a bacterial culture medium is provided in the form of a paper, such as a paper strip or a blotting paper or a blotting paper strip, impregnated with the bacterial-sensitive indicator. Alternatively, paper-based diagnostic methods, in the context of this disclosure, may refer to methods wherein the bacterial-sensitive indicator is provided in the form of a first paper, such as a first paper strip or a first blotting paper or a first blotting paper strip, impregnated with the bacterial-sensitive indicator, and a bacterial culture medium is provided in the form of a second paper, such as a second paper strip or a second blotting paper or a second blotting paper strip, impregnated with the bacterial culture medium.

Paper-based diagnostic methods are described, for example, in EP 4 109 101 A1. EP 4 109 101 A1 is incorporated herein in its entirety by reference.

The inventors have realized that the sensitivity and specificity of paper-based diagnostic methods are, to a surprisingly high degree, compromised by the presence of contaminants other than the bacteria in the urine sample being subject to the diagnostic method. The effect on the sensitivity is particularly pronounced.

Without wishing to be bound by any theory, the inventors hypothesize that the surprisingly strong effect of the contaminants on the sensitivity and specificity of the paper-based diagnostic methods is caused by colored and/or optically strongly absorbing contaminants in the urine. These contaminants affect the optical inspection and analysis of the bacterial-sensitive indicator which has been exposed to the urine. The colored and/or optically strongly absorbing contaminants give rise to an undesirable "background" color change of the bacterial-sensitive indicator, this background color change occurring due to the presence of the colored and/or optically strongly absorbing contaminants, independent of whether bacteria are present in the fluid (e.g., urine) subject to the diagnostic method. The background color change reduces the sensitivity, in particular, since it exhibits strong sample-to-sample variations, in a sense that the concentration of the colored and/or optically strongly absorbing contaminants differs significantly between different urine samples. The detection of any indication of bacteria (e.g., color change) by the bacterial-sensitive indicator on top of this sample-to-sample varying background color change becomes challenging.

Backwashing, as made possible by the device according to this disclosure, reduces the presence of contaminants at the bacterial-sensitive indicator significantly. It thus significantly improves the sensitivity of diagnostic methods that the bacterial-sensitive indicator is used in, and in particular of paper-based diagnostic methods and of diagnostic methods applied to urine.

Specifically, in the diagnostics of urinary tract infections according to the state-of-the-art, urine culture is the preferred technique to be applied. This method requires laboratory facilities and takes several days to yield results. Urine dipstick tests are faster but lack the sensitivity and specificity needed. Moreover, they cannot differentiate between bacterial infections. Using the device according to this disclosure, paper-based diagnostic methods of urinary tract infections are made possible or are significantly improved. In particular, the device is well-suited for point-of-care diagnostics using paper-based diagnostic methods, which is not feasible with conventional diagnostics of urinary tract infections, such as urine culture.

According to an embodiment, the device further comprises an additional filter adapted to permit the bacteria contained in a fluid to pass through the additional filter when the fluid containing the bacteria passes through the additional filter. The additional filter may be arranged between the first position and the inlet.

Respective embodiments further reduce the amount of contaminants, such as colored and/or optically strongly absorbing contaminants, that reach the bacterial-sensitive indicator, by filtering out larger particles. In particular, the additional filter filters out oversized contaminants, i.e., contaminants larger than the bacteria to be detected and/or larger than pores of the additional filter, respectively.

The additional filter may have pore sizes exceeding 1 µm or exceeding 2 µm or exceeding 3 µm.

In the context of this disclosure, pore sizes may refer to diameters of pores, for example to an average diameter of the pores.

Respective embodiments are particularly beneficial for detecting bacteria such as Escherichia coli bacteria, which have a diameter of around 1 µm and which pose a common origin of urinary tract infections.

According to an embodiment, the device further comprises a fluid absorber arranged below the first position.

The fluid absorber may be adapted to provide, when the fluid enters the device through the inlet, a capillary suction force acting on the fluid, said capillary suction force being directed from the inlet towards the filter and the fluid absorber.

The fluid absorber may comprise or may be composed of cotton.

Positioning the fluid absorber below the first position ensures efficient fluid management by utilizing capillary action to direct the fluid towards the filter. This arrangement enhances the device's ability to process the fluid quickly and effectively, reducing the likelihood of fluid stagnation and ensuring that bacteria are efficiently retained by the filter.

The capillary suction force aids in drawing the fluid from the inlet towards the filter and the fluid absorber itself. The capillary suction force is advantageous as it promotes the efficient movement of fluid through the device, ensuring that the fluid reaches the filter where bacteria can be retained. This mechanism reduces the need for additional mechanical components to move the fluid, simplifying the device's design and potentially lowering manufacturing costs.

Cotton is highly absorbent, which ensures efficient fluid uptake and retention. Additionally, cotton is a natural, biocompatible material that does not introduce contaminants, making it suitable for sensitive bacterial detection applications.

According to some embodiments, the device comprises, below the second position, an additional inlet for introducing, when the filter is at the second position, an additional fluid through the additional inlet and through the filter towards the bacterial-sensitive indicator.

Having an additional inlet below the second position provides the advantage of enabling the introduction of a secondary fluid into the system, which can be used to backwash the filter and extract the bacteria, or to inoculate the bacteria in the secondary fluid, respectively.

The device may further comprise a fluid reservoir comprising the additional fluid, and a fluid-introduction device adapted to drive the additional fluid from the fluid reservoir through the additional inlet.

This reservoir ensures that there is a sufficient supply of the additional fluid required for the operation of the device.

The provision of the reservoir with the additional fluid further ensures that a suitable (additional) fluid, such as phosphate buffered saline, is provided for backwashing the filter and for suspending or inoculating the bacteria.

According to an embodiment, the device further comprises a syringe comprising the fluid reservoir and providing the fluid-introduction device.

Respective embodiments allow for precise control over the volume of fluid introduced into the device, ensuring that the correct amount is used for accurate bacterial detection. The syringe also facilitates easy handling and transport of the fluid, minimizing the risk of contamination. The device and a method using the device thus benefit from a controlled and efficient method of fluid delivery.

According to an embodiment, the device comprises a separation layer arranged between the first position and the additional filter.

The separation layer allows for more controlled movement of the filter between positions, which can lead to more precise operation of the device.

According to an embodiment, the separation layer is adapted to provide a sliding layer that reduces sticking of the filter to the additional filter when the filter moves from the first position to the second position.

In the context of this disclosure, the separation layer may refer to a distinct layer or coating applied between the filter and the additional filter. This layer may be specifically designed to facilitate smooth movement by reducing friction and preventing adhesion between the two filters (i.e. between the filter and the additional filter).

Reducing sticking of the filter to the additional filter when the filter moves from the first position to the second position ensures that the filter can move freely without obstruction. This enhances the reliability and efficiency of the device, as it prevents potential blockages or delays in the detection process.

According to an embodiment, the device further comprises an observation window arranged on the device such that the bacterial-sensitive indicator is visible from outside of the device through the observation window.

The observation window may refer to a transparent or translucent section integrated into the device's housing. Alternatively, the observation window may be implemented as an opening or an aperture in the device's housing.

According to an embodiment, the observation window is arranged above the bacterial-sensitive indicator.

According to an embodiment, the bacterial-sensitive indicator comprises a chromogenic substance. Alternatively, or in addition, the bacterial-sensitive indicator may comprise a fluorogenic substance.

In the context of this disclosure, a bacterial-sensitive indicator that comprises a chromogenic substance may refer to a component that changes color in response to the presence of bacteria. This change in color is typically due to a chemical reaction between the chromogenic substance and specific enzymes or metabolites produced by the bacteria.

A chromogenic substance provides a visual indication of bacterial presence, which can be easily observed without the need for specialized equipment. This feature enhances the usability of the device, in particular in point-of-care diagnostics.

In the context of this disclosure, a bacterial-sensitive indicator that comprises a fluorogenic substance may refer to a component that emits fluorescence when exposed to light, in particular to blue or UV light, in the presence of bacteria. This fluorescence is typically due to a reaction between the fluorogenic substance and bacterial enzymes or metabolites.

A fluorogenic substance offers a sensitive and precise method for detecting bacteria, as fluorescence can be quantified using fluorometers. This feature allows for the detection of low concentrations of bacteria, improving the sensitivity and reliability of the device.

The bacterial-sensitive indicator may comprise a paper, said paper comprising the chromogenic substance and/or the fluorogenic substance.

Using paper as the medium for the chromogenic or fluorogenic substances allows for a simple and cost-effective design.

The bacterial-sensitive indicator may comprise or may be a redox indicator and/or an enzymatic indicator.

A redox indicator may refer to a chemical compound that undergoes a distinct color change when it is reduced or oxidized. This property allows indicating the presence of bacteria, which can alter the redox state of the environment through metabolic activities.

Utilizing a redox indicator provides a clear and immediate visual signal of bacterial presence, enhancing the device's ability to detect bacteria quickly and accurately.

In the context of this disclosure, an enzymatic indicator may refer to a substance that reacts with specific enzymes produced by bacteria, resulting in a detectable change, such as a color shift.

An enzymatic indicator provides a highly specific method of detecting bacteria, as it can be designed to react with enzymes unique to certain bacterial species. This specificity can reduce false positives and increase the accuracy of bacterial detection.

The bacterial-sensitive indicator may comprise a paper, said paper comprising the redox indicator and/or the enzymatic indicator.

According to an embodiment, the device further comprises a bacterial culture medium adapted to support bacterial growth, said bacterial culture medium being arranged above the second position and in liquid communication with the bacterial-sensitive indicator.

In the context of this disclosure, a bacterial culture medium adapted to support bacterial growth may refer to a substrate or solution that provides the necessary nutrients and environmental conditions conducive for the proliferation of bacteria. This medium typically contains nutrients such as carbon, nitrogen, vitamins, and minerals that support bacterial metabolism and growth.

The bacterial culture medium, when combined with the features of the aspect, facilitates the growth of bacteria retained by the filter after it has been moved to the second position. This growth enlarges the number of bacteria, resulting in an enhanced probability for the bacterial-sensitive indicator to detect and signal the presence of bacteria.

The device may further comprise a second paper impregnated with the bacterial culture medium, the second paper being arranged above the second position and in liquid communication with the bacterial-sensitive indicator.

This second paper is designed to work in conjunction with the bacterial-sensitive indicator to enhance the detection of bacteria, as it can increase the sensitivity and accuracy of bacterial detection.

The device may further comprise an antibiotic drug, said antibiotic drug being arranged above the second position and in liquid communication with the bacterial-sensitive indicator.

The device may further comprise a third paper impregnated with the antibiotic drug, the third paper being arranged above the second position and in liquid communication with the bacterial-sensitive indicator.

Respective embodiments allow assessing a possible resistance of the bacteria to the antibiotic drug. In respective embodiments, typically, the antibiotic drug (or the third paper impregnated with the antibiotic drug, respectively) is arranged above a first section of the bacterial culture medium (or of the second paper impregnated with the bacterial culture medium, respectively), preferably in liquid communication therewith. A second section of the bacterial culture medium (or of the second paper impregnated with the bacterial culture medium, respectively) is not overlain by the antibiotic drug (or the third paper impregnated with the antibiotic drug, respectively).

Consequently, if the bacteria are nonresistant to the antibiotic drug, bacterial growth and detection is inhibited in or above the first section. To the contrary, if the bacteria are resistant to the antibiotic drug, bacterial growth and detection is not inhibited in or above the first section.

The bacterial-sensitive indicator is arranged above both the first section of the bacterial culture medium and the second section of the bacterial culture medium. By comparing the reaction of the bacterial-sensitive indicator above both the first section of the bacterial culture medium and above the second section of the bacterial culture medium, a possible resistance of the bacteria to the antibiotic drug is detected.

According to an embodiment, the filter has pore sizes smaller than 1 µm or smaller than 0.8 µm.

Respective embodiments are particularly beneficial for detecting bacteria such as Escherichia coli bacteria, which have a diameter of around 1 µm and which pose a common origin of urinary tract infections.

The device may further comprise a sliding member slidably mounted to the device, wherein the filter is connected to the sliding member such that the filter moves together with the sliding member.

The sliding member and the filter connected thereto may be adapted such that the filter moves between the first position and the second position as it moves together with the sliding member.

In the context of this disclosure, a sliding member slidably mounted to the device may refer to a component that is designed to move along a predefined path within the device, e.g., linearly. This sliding member facilitates the movement of the filter attached to it, ensuring precise positioning and alignment. The sliding member ensures that the filter can be accurately positioned under the inlet and the bacterial-sensitive indicator. This precise movement enhances the reliability and efficiency of the bacterial detection process.

In respective embodiments, said sliding member may be slidable along the horizontal direction.

Alternatively, or in addition, the sliding member may be arranged partially inside of the device and partially outside of the device; in particular, when the filter is at the second position or when the filter is at the first position and when the filter is at the second position.

Having the first position and/or the second position inside the device ensures that the entire bacterial detection process occurs within a controlled environment, minimizing exposure to external contaminants. This internal arrangement can lead to more accurate and reliable detection results.

The bacterial-sensitive indicator may be arranged inside the device. Alternatively, or in addition, the first position and/or the second position may be inside the device.

Respective embodiments reduce the risk of contamination and external interference.

The bacteria may be Escherichia coli bacteria.

In particular, said filter may be adapted to retain respective bacteria (i.e., with diameters of around 1 µm, e.g. in a range from 1 micrometres to 1.5 micrometres), and the additional filter may be adapted to let them pass.

The fluid may be urine.

The device may further comprise a support arranged horizontally adjacent to the fluid absorber, the support comprising an upper surface adapted to support the filter at the second position.

According to a second aspect of the disclosure, a device for detecting a biological probe contained in a fluid is provided. The device comprises an inlet arranged on the device. The inlet is adapted to permit the fluid to enter the device therethrough. The device comprises a filter adapted to retain the biological probe contained in the fluid, and an indicator sensitive to the biological probe. The indicator sensitive to the biological probe is adapted to indicate a presence of the biological probe. The indicator sensitive to the biological probe is arranged at a position horizontally offset from a position of the inlet. The filter is arranged moveably between a first position below the inlet and a second position below the indicator sensitive to the biological probe.

The biological probe may comprise at least one of the following or may be one of the following: bacteria and fungi (for example, mold).

The indicator sensitive to the biological probe may comprise at least one of the following or may be one of the following: a bacterial-sensitive indicator and a fungi-sensitive indicator.

According to different embodiments, the indicator sensitive to the biological probe of the device according to the second aspect may be formed with one, a combination of, or all the optional features described above in the context of the antibiotic drug of the device according to the first aspect.

According to different embodiments, the device according to the second aspect is similar to the device described above in the context of the first aspect of this disclosure. In other words, according to different embodiments, the device according to the second aspect may be formed with one, a combination of, or all the optional features described above in the context of the device according to the first aspect.

For example, the device according to the second aspect may comprise a bioactive substance, said bioactive substance being arranged above the second position and in liquid communication with the indicator sensitive to the biological probe.

The bioactive substance may comprise at least one of the following or may be one of the following: an antibiotic drug and an antifungal drug.

According to different embodiments, the bioactive substance of the device according to the second aspect may be formed with one, a combination of, or all the optional features described above in the context of the antibiotic drug of the device according to the first aspect.

Although the device is described with reference to the exemplary detection of bacteria (i.e., using a bacterial-sensitive indicator), the device can be applied similarly to the detection of fungi (i.e., using a fungi-sensitive indicator), for example, in the context of detecting fungi (such as mold) in the context of water analysis or detecting fungi in the context of medical analysis. A respective device achieves similar technical effects and advantages in the context of detecting fungi as the device according to the first aspect achieves in the context of detecting bacteria.

According to an aspect of the disclosure, a method for extracting bacteria contained in urine is provided. The method comprises flowing the urine through a filter along a first flow direction from a first side of the filter to a second side of the filter opposite to the first side. The filter retains the bacteria contained in the urine on the first side of the filter. Thereafter, the method comprises flowing an additional fluid through the filter from the second side of the filter to the first side of the filter, thus inoculating the bacteria in the additional fluid flown to said first side.

The filter applied in the method may be similar to the filter described above in the context of the device according to the first aspect of the disclosure. In other words, the filter applied in the method may exhibit one or all the characteristics described above in the context of the filter of the device.

The method implements a backwashing technique for extracting bacteria from urine, with the advantages laid out above.

In the process step of flowing the urine through the filter, an additional filter adapted to permit the bacteria contained in the urine to pass therethrough may be arranged on the first side of the filter, and the urine may be flown through the additional filter and through the filter.

The additional filter applied in the method may be similar to the filter described above in the context of the device according to the first aspect of the disclosure. In other words, the additional filter applied in the method may exhibit one or all the characteristics described above in the context of the additional filter of the device.

The additional filter applied in the method achieves the advantages described above in the context of the additional filter of the device.

According to some embodiments, in the process step of flowing the additional fluid through the filter from the second side of the filter to the first side of the filter, the additional fluid is not flown through the additional filter.

Respective embodiments prevent that contaminants retained in the additional filter are backwashed with the additional fluid, or that they are inoculated in the additional fluid, respectively.

The method may further comprise in between the process steps of flowing the urine through the filter and flowing the additional fluid through the filter, removing the additional filter from the first side of the filter.

In the context of this disclosure, "removing the additional filter from the first side of the filter" may refer to the step of physically detaching or taking away an additional filter that is positioned on the first side of the primary filter.

This ensures that the bacteria retained on the first side of the filter are effectively inoculated into the additional fluid, without contaminations from the material retained in the additional filter.

According to an embodiment, the process step of flowing the urine through the filter comprises flowing the urine through an at-least-three-way valve downstream from the filter from a first port of the at-least-three-way valve to a second port of the at-least-three-way valve; and the process step of flowing the additional fluid through the filter from the second side of the filter to the first side of the filter comprises flowing the additional fluid through the at-least-three-way valve from a third port of the at-least-three-way valve to the first port of the at-least-three-way valve, the at-least-three-way valve being arranged upstream from the filter in the respective process step.

The at-least-three-way valve may refer to a valve comprising at least three ports, adapted to provide a fluid-connection between two of said at least three ports, the respective two of said at least three ports being selectable. In particular, the at least three ports may be exactly three ports.

The use of the at-least-three-way valve downstream from the filter makes the method more streamlined and efficient. Respective embodiments allow for performing the method faster, which may be particularly relevant in a clinical environment. The at-least-three-way valve may be provided with one (or some) of the elements of the method provided (and/or preprepared) at one (or each) of the respective elements on one of the ports. For example, the respective element(s) may be provided/arranged on the ports by clinical personal during times when clinical personal would otherwise be idling. The provided/preprepared elements make the equipment available for quick performance of the method whenever the diagnostic technique needs to be performed.

For example, the at-least-three-way valve may be provided (and/or preprepared) with the additional fluid at a port thereof, speeding up the method even further.

The at-least-three-way valve also allows for precise management of fluid flow in both directions, ensuring thorough filtration and effective inoculation of bacteria into the additional fluid. This results in a more reliable and streamlined method for extracting bacteria from urine samples.

The method may further comprise, in between the process steps of flowing the urine through the filter and flowing the additional fluid through the filter, flowing a purge liquid through the filter along the first flow direction.

A volume of the purge liquid flown through the filter may exceed a volume of the urine flown through the filter.

According to an embodiment, a collection reservoir is arranged on the second port of the at-least-three-way valve, and wherein the collection reservoir receives the urine flown through the at-least-three-way valve from the first port of the at-least-three-way valve to the second port of the at-least-three-way valve.

For example, the at-least-three-way valve may be provided (and/or preprepared) with the collection reservoir at a port thereof, speeding up the method even further.

The collection reservoir may comprise or may be a collection bag.

The method may further comprise, after inoculating the bacteria, performing a bacterial cultivation on the bacteria.

In the context of this disclosure, "performing a bacterial cultivation on the bacteria" may refer to the process of growing and multiplying the bacteria in a controlled environment, such as a laboratory setting, using specific nutrients and conditions that promote bacterial growth, with the advantages described above for the cell culture in the context of the device according to the first aspect.

The method may further comprise, after inoculating the bacteria, applying a bacterial-sensitive indicator to the bacteria to identify the bacteria.

Applying a bacterial-sensitive indicator to the bacteria after inoculation allows for the identification of the bacteria that have been successfully retained and inoculated.

The method may further comprise, after inoculating the bacteria, applying an antibiotic drug to the bacteria.

Applying an antibiotic drug to the bacteria can help in determining the susceptibility of the bacteria to specific antibiotics, which helps in selecting the appropriate treatment for bacterial infections. This step can also aid in the rapid identification of effective antibiotics, thereby potentially reducing the time required for treatment decisions.

According to some embodiments, the method comprises, after inoculating the bacteria, performing a bacterial cultivation on the bacteria, and applying a bacterial-sensitive indicator to the bacteria to identify the bacteria.

According to some embodiments, the method comprises after inoculating the bacteria, performing a bacterial cultivation on the bacteria and applying an antibiotic drug to the bacteria, and applying a bacterial-sensitive indicator to the bacteria to identify the bacteria.

The process step of flowing the urine through the filter may comprise driving the urine through the filter using a first syringe.

The process step of flowing the additional fluid through the filter may comprise driving the additional fluid through the filter using a second syringe.

Using a syringe to drive the urine and/or the additional fluid through the filter provides a simple and effective means of applying the necessary pressure to ensure the urine and/or the additional fluid, respectively, passes through the filter medium. This method is advantageous because it allows for precise control over the flow rate and the amount of fluid, which can improve the reproducibility and accuracy of the method.

The bacterial-sensitive indicator of the method may be impregnated in a paper.

The bacterial cultivation of the method may be performed using a bacterial culture medium impregnated in a second paper.

The antibiotic drug of the method may be impregnated in a third paper.

According to an aspect of the disclosure, a method for extracting a biological probe contained in a fluid is provided. The method comprises flowing the fluid through a filter along a first flow direction from a first side of the filter to a second side of the filter opposite to the first side. The filter retains the biological probe contained in the fluid on the first side of the filter. Thereafter, the method comprises flowing an additional fluid through the filter from the second side of the filter to the first side of the filter, thus inoculating the biological probe in the additional fluid flown to said first side.

According to different embodiments, a respective method is similar to the method for extracting bacteria contained in urine described above (however, with respect to the biological probe instead of with respect to the bacteria, and with respect to the fluid instead of with respect to the urine). In other words, according to different embodiments, the respective method may be performed with one, a combination of, or all the optional features described above in the context of the method for extracting bacteria contained in urine (however, with respect to the biological probe instead of with respect to the bacteria, and with respect to the fluid instead of with respect to the urine).

The biological probe may comprise at least one of the following or may be one of the following: bacteria and fungi (for example, mold).

Although the method is described with reference to extracting bacteria from urine (e.g., using a bacterial-sensitive indicator), the method can be applied similarly to extracting fungi from fluids (i.e., using a fungi-sensitive indicator), for example, in the context of detecting fungi (such as mold) in the context of water analysis or detecting fungi in the context of medical analysis. A respective method achieves similar technical effects and advantages as the method for extracting bacteria from urine.

A further aspect relates to a use of the device according to any of the embodiments described above (e.g., the device according to the second aspect) for detecting a biological probe contained in the fluid.

### LIST OF FIGURES

In the following, a detailed description of the present disclosure and examples thereof are given with reference to the figures, wherein
Fig. 1 shows a device according to an embodiment, with the filter in the first position;
Fig. 2 shows the device according to the embodiment of Fig. 1, with the filter in the second position;
Fig. 3a and Fig. 3b illustrate a device according to another embodiment;
Fig. 4 to Fig. 8 each illustrates a device according to another, different embodiment;
Fig. 9a, Fig. 9b illustrate a device according to another embodiment;
Fig. 10 illustrates a method according to an embodiment;
Fig. 11a, Fig. 11b illustrate a method according to another embodiment;
Fig. 12 illustrates a further, optional process step of the method;
Fig. 13a to Fig. 13c illustrate a method according to another embodiment;
Fig. 14 illustrates a method according to another embodiment; and
Fig. 15 illustrates a method according to another embodiment.

### DESCRIPTION OF EXAMPLES

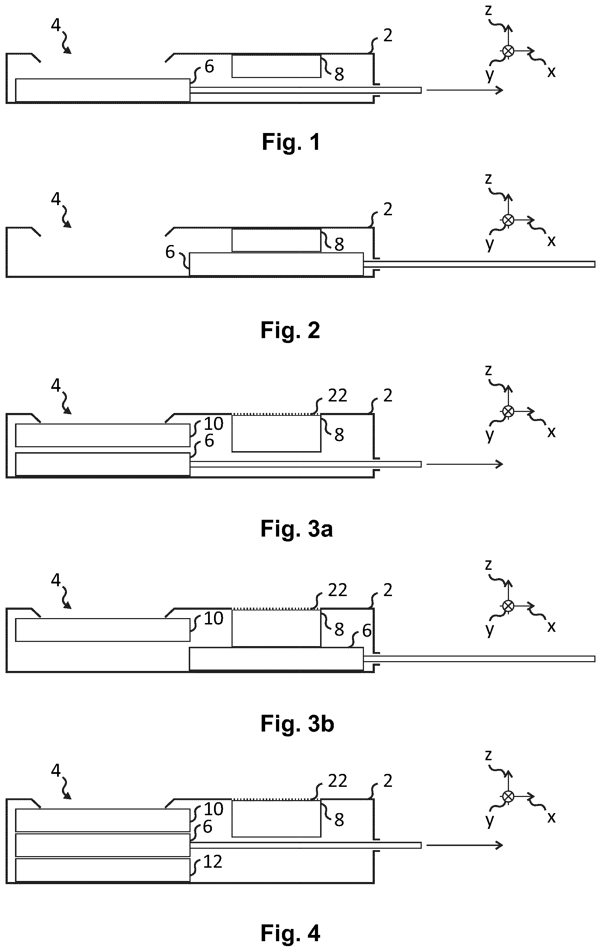

Fig. 1 gives a cross-sectional view of a device 2 for detecting bacteria in a fluid. The device 2 comprises an inlet 4, a filter 6, and a bacterial-sensitive indicator 8. The inlet 4 is positioned to allow fluid entry into the device 2. The filter 6 is designed to retain bacteria present in the fluid, and the bacterial-sensitive indicator 8 is positioned horizontally offset from the inlet 4. The filter 6 is shown in a position that aligns with the inlet 4, indicating its first position.

For reference, the figure also indicates a coordinate system with axes x, y, and z. In the context of this disclosure, the z direction is also referred to as the vertical direction. The x-y-plane is also referred to as a horizontal plane or a lateral plane. Notably, this terminology and terms relating thereto, such as "above" or "below", shall indicate the positions and orientations of the elements of the device 2 relative to each other, as also indicated in the figures. It is not meant to imply any orientation relative to an external coordinate system, such as an external coordinate system defined by the gravitational field of the earth.

The inlet 4 is an opening in a housing (not indicated in Fig. 1) that permits the fluid to enter the device 2, or its housing, respectively. The housing is typically made from a material that is resistant to the fluid being tested, such as plastic or metal.

The housing and the inlet 4 serve to provide a controlled entry point for the fluid, ensuring that the fluid flows directly to the filter 6 without exposure to external contaminants.

The filter 6 is designed to retain (trap) bacteria from the fluid. It is composed of a material with pore sizes smaller than the bacteria to be extracted and/or detected. In the depicted embodiment, the filter 6 is made from polyether sulfone and has pore sizes of 0.45 micrometres.

The filter 6 configured this way allows fluid, in particular urine, to pass through while retaining bacteria on its surface. This applies, in particular, to Escherichia coli bacteria, which are a typical source of urinary tract infections, and which are rod shaped bacteria with a diameter of around 1 micrometres, more specifically, often slightly above one micrometre and below two micrometres.

The filter 6 can thus have pore sizes in the range from 0.1 µm to 1 µm, preferably in the range from 0.2 µm to 0.8 µm. Pore sizes smaller than 0.1 µm are possible, but have the disadvantage of a significantly increased flow resistance of the filter 6, which undesirably slows down the process of flowing the fluid therethrough.

In alternative embodiments, a membrane filter made from polycarbonate or cellulose acetate is applied, or a filter comprising nylon, polyester, or glass fiber; or a microfiltration membrane, a nanofiber filter, or a ceramic filter.

The bacterial-sensitive indicator 8 is positioned to detect the presence of bacteria retained by the filter 6.

For this purpose, a material of the bacterial-sensitive indicator 8 is adapted to react with bacterial enzymes or metabolites, producing a visible color change or fluorescence. The bacterial-sensitive indicator 8 is often integrated into a paper or strip.

In the depicted embodiment, the bacterial-sensitive indicator 8 is provided in the form of filtration paper (thickness: 1.5 mm) impregnated with a chromogenic substance.

According to different embodiments (not shown), said material of the bacterial-sensitive indicator 8 comprises at least one of: a chromogenic substance, a fluorogenic substance, a redox indicator and an enzymatic indicator.

Said material of the bacterial-sensitive indicator 8, according to different embodiments (not shown), is in the form of a gel, a liquid reagent, or an electronic sensor that provides a digital readout.

The device 2 has been described in the context of the detection of bacteria contained in a fluid, such as urine. However, with minor modifications, the device 2 can be beneficially applied to detect fungi such as mold, e.g., in the context of water analysis or medical diagnostics. A respective, modified device (not shown) comprises, instead of the bacterial-sensitive indicator 8, an indicator sensitive to the biological probe to be detected, such as an indicator sensitive to fungi. Instead of the filter 6 adapted to retain the bacteria 36, the device 2 comprises a filter adapted to retain the biological probe (in particular, the fungi).

Fig. 2 shows another cross-sectional view of the device 2 depicted in Fig. 1.

In Fig. 2, the filter 6 is in the second position below the bacterial-sensitive indicator 8.

This configuration allows the bacteria retained on the filter 6 to interact with the bacterial-sensitive indicator 8, facilitating detection.

The movement of the filter 6 to the second position is achieved through a sliding mechanism or a pivoting arm.

Fig. 3a, Fig. 3b give cross-sectional views of a device 2 for detecting bacteria in a fluid according to another embodiment.

The embodiment of Fig. 3a, Fig. 3b is similar to the embodiment of Fig. 1 and Fig. 2. Similar elements are indicated with identical reference numerals. To avoid repetition, for a detailed description of these elements, reference is made to the corresponding description above in the context of the foregoing embodiment.

Fig. 3a shows the device 2 with the filter 6 in the first position, and Fig. 3b shows the device 2 with the filter 6 in the second position.

As a modification over the embodiment of Fig. 1, Fig. 2, the device 2 of Fig. 3a, Fig. 3b comprises an additional filter 10 positioned between the inlet 4 and the filter 6.

As a modification over the embodiment of Fig. 1, Fig. 2, the device 2 of Fig. 3a, Fig. 3b comprises an observation window 22 above the bacterial-sensitive indicator 8.

As compared to the embodiment of Fig. 1 and Fig. 2, the embodiment of Fig. 3a, Fig. 3b is thus formed with multiple modifications. According to different embodiments (not shown), the device according to the embodiment of Fig. 1 and Fig. 2 or of any of the other embodiments described in this disclosure is formed with only one or any combination of the modifications described in the context of Fig. 3a, Fig. 3b.

The structure and material composition of the additional filter 10 is similar to the structure and material composition of the filter 6, but the additional filter 10 has larger pore sizes.

In the depicted embodiment, the additional filter 10 is made from polyether sulfone and has pore sizes of 5 micrometres.

The additional filter 10 is thus adapted to allow the bacteria and the fluid to pass through the additional filter 10. This applies, in particular, to the Escherichia coli bacteria described above.

For this purpose, the additional filter 10 generally has pore sizes larger than 1 µm, preferably larger than 2 µm or larger than 3 µm.

The observation window 22 is a transparent section of the device 2, or of its housing, respectively, that allows for visual inspection of the bacterial-sensitive indicator 8.

In the depicted embodiment, the observation window 22 is an aperture (opening) in the housing of the device 2, located above the bacterial-sensitive indicator 8.

In alternative embodiments (not shown), the observation window 22 comprises an at least partially transparent material, for example glass or polycarbonate.

Fig. 4 gives a cross-sectional view of a device 2 for detecting bacteria in a fluid according to another embodiment.

The embodiment of Fig. 4 is similar to the embodiment of Fig. 3a, Fig. 3b. Similar elements are indicated with identical reference numerals. To avoid repetition, for a detailed description of these elements, reference is made to the corresponding description above in the context of the foregoing embodiments.

Fig. 4 shows the device 2 with the filter 6 in the first position. The device 2 with the filter 6 in the second position is not depicted, but is similar to the embodiment of Fig. 3b, yet with the modifications of the embodiments of Fig. 4 described in the following.

As a modification over the embodiment of Fig. 3a, Fig. 3b, the device 2 of Fig. 4 comprises a fluid absorber 12 positioned below the first position of the filter 6.

According to alternative embodiments (not shown), the device 2 according to the embodiment of Fig. 1 and Fig. 2 or of any of the other embodiments described in this disclosure is formed with the fluid absorber 12 described in the context of Fig. 4.

In the depicted embodiment, the fluid absorber 12 comprises a lower layer, being an absorbent pad made from cotton, and an upper layer, being blotting paper. In alternative embodiments (not shown), the fluid absorber 12 comprises only one of those layers.

A material alternative to the cotton and the blotting paper mentioned above can be used for the fluid absorber 12, as long as the material is adapted to maximize surface area and capillary action, ensuring efficient fluid absorption. Exemplary materials include sponge, foam, or superabsorbent polymers.

The advantage of the fluid absorber 12 is that it facilitates fluid flow through the filter 6 (and the additional filter 10), enhancing the device's 2 efficiency in capturing bacteria. By providing a consistent suction force, the fluid absorber 12 ensures that the fluid is evenly distributed across the filter surface, maximizing bacterial retention. The fluid absorber 12 thus ensures efficient fluid flow, enhancing the device's 2 overall performance.

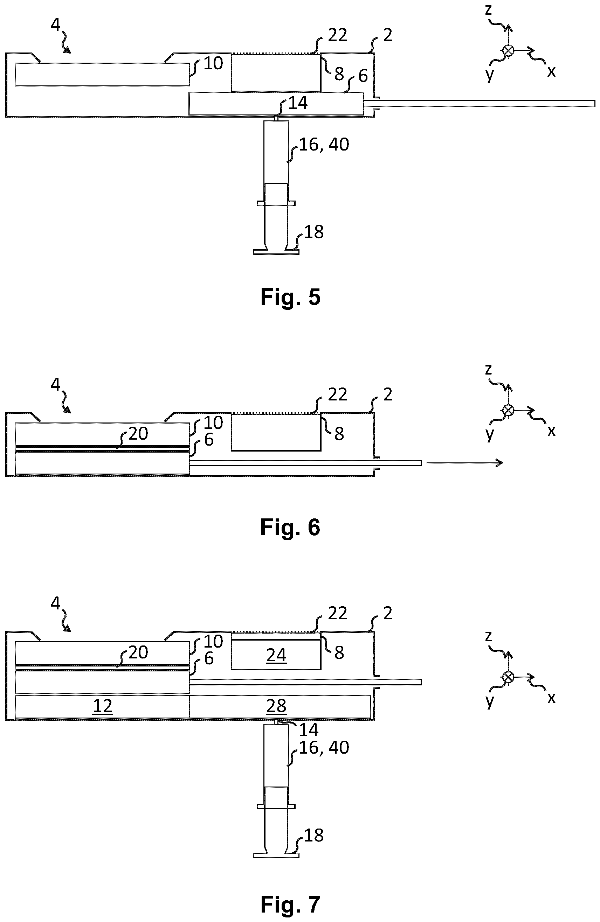

Fig. 5 gives a cross-sectional view of a device 2 for detecting bacteria in a fluid according to another embodiment.

The embodiment of Fig. 5 is similar to the embodiment of Fig. 3a, Fig. 3b. Similar elements are indicated with identical reference numerals. To avoid repetition, for a detailed description of these elements, reference is made to the corresponding description above in the context of the foregoing embodiments.

The device 2 according to the embodiment of Fig. 5 is optionally formed with the fluid absorber 12 (not shown in Fig. 5).

Fig. 5 shows the device 2 with the filter 6 in the second position. The device 2 with the filter 6 in the first position is not depicted, but is similar to the embodiment of Fig. 3a, yet with the modifications of the embodiments of Fig. 5 described in the following.

As a modification over the embodiment of Fig. 3a, Fig. 3b, the device 2 of Fig. 5 comprises an additional inlet 14.

As a modification over the embodiment of Fig. 3a, Fig. 3b, the device 2 of Fig. 5 comprises a fluid reservoir 16.

As a modification over the embodiment of Fig. 3a, Fig. 3b, the device 2 of Fig. 5 comprises a fluid-introduction device 18.

As compared to the foregoing embodiments, the embodiment of Fig. 5 is thus formed with multiple modifications. According to different embodiments (not shown), the device 2 according to the embodiment of Fig. 1 and Fig. 2, of Fig. 3a, Fig. 3b, of Fig. 4, or of any of the other embodiments described in this disclosure is formed with only one or any combination of the modifications described in the context of Fig. 5.

The additional inlet 14 is used for introducing an additional fluid 40 through the filter 6 towards the bacterial-sensitive indicator 8.

In the depicted embodiment, a fluid reservoir 16 is connected to the additional inlet 14.

In the depicted embodiment, the fluid reservoir 16 comprises the additional fluid 40 in the exemplary form of phosphate buffered saline.

In alternative embodiments (not shown), the device 2 is provided only with the additional inlet 14, but without the additional fluid 40. In alternative embodiments (not shown), the device 2 is provided only with the additional inlet 14, but without the fluid reservoir 16 and the additional fluid 40, which are provided separately.

In the depicted embodiment, a fluid-introduction device 18 in the form of a syringe is further provided. More specifically, in the depicted embodiment, the syringe combines the fluid reservoir 16 and the fluid-introduction device 18.

In alternative embodiments (not shown), the fluid reservoir 16 is provided, but not necessarily the fluid-introduction device 18.

The additional fluid 40 is introduced through the additional inlet 14, flowing through the filter 6 and inoculating the bacteria in the bacterial-sensitive indicator 8. The syringe providing the fluid reservoir 16 and fluid-introduction device 18 ensures controlled delivery of additional fluids 40 for bacterial analysis.

Fig. 6 gives a cross-sectional view of a device 2 for detecting bacteria in a fluid according to another embodiment.

The embodiment of Fig. 6 is similar to the embodiment of Fig. 3a, Fig. 3b. Similar elements are indicated with identical reference numerals. To avoid repetition, for a detailed description of these elements, reference is made to the corresponding description above in the context of the foregoing embodiments.

Fig. 6 shows the device 2 with the filter 6 in the first position. The device 2 with the filter 6 in the second position is not depicted, but is similar to the embodiment of Fig. 3b, yet with the modifications of the embodiments of Fig. 6 described in the following.

As a modification over the embodiment of Fig. 3a, Fig. 3b, the device 2 of Fig. 6 comprises a separation layer 20, providing a sliding layer.

According to different embodiments (not shown), the device 2 according to the embodiment of Fig. 1 and Fig. 2, of Fig. 3a, Fig. 3b, of Fig. 4, Fig. 5, or of any of the other embodiments described in this disclosure is formed with such separation layer 20.

The separation layer 20 provides a sliding layer that reduces sticking of the filter 6 to the additional filter 10 when the filter 6 moves from the first position to the second position. The separation layer 20 ensures smooth movement of the filter 6, preventing mechanical issues during operation.

In the depicted embodiment, the separation layer 20 is a layer of paper, more specifically, of filter paper.

Fig. 7 gives a cross-sectional view of a device 2 for detecting bacteria in a fluid according to another embodiment.

The embodiment of Fig. 7 is similar to the foregoing embodiments. Similar elements are indicated with identical reference numerals. To avoid repetition, for a detailed description of these elements, reference is made to the corresponding description above in the context of the foregoing embodiments.

The device 2 with the combination of elements according to the embodiment of Fig. 7 provides a particularly beneficial embodiment.

The device 2 according to the embodiment of Fig. 7 further comprises, optionally, a support 28. The support 28 is arranged horizontally adjacent to the fluid absorber 12 and supports the filter 6 at the second position. It typically has a flat upper surface. The support 28 ensures stable positioning of the filter 6 during fluid introduction and bacterial analysis. According to alternative embodiments (not shown), the support 28 is not present.

According to alternative embodiments (not shown) the device 2 according to any of the other embodiments of this disclosure is formed with the support 28.

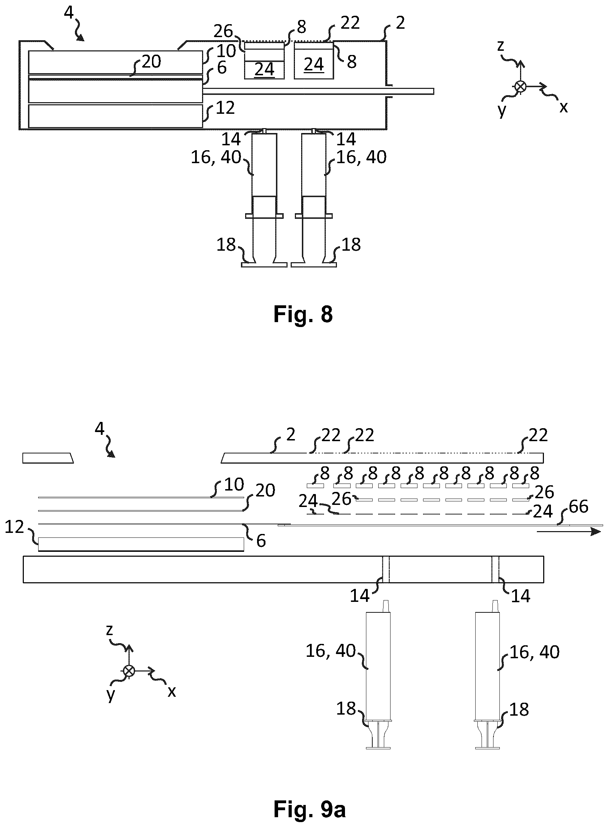

Fig. 8 gives a cross-sectional view of a device 2 for detecting bacteria in a fluid according to another embodiment.

The embodiment of Fig. 8 is similar to the embodiment of Fig. 7. Similar elements are indicated with identical reference numerals. To avoid repetition, for a detailed description of these elements, reference is made to the corresponding description above in the context of the foregoing embodiments.

Fig. 8 shows the device 2 with the filter 6 in the first position. The device 2 with the filter 6 in the second position is not depicted, but is similar to the embodiment of Fig. 3b, yet with the modifications of the embodiments of Fig. 8.

As a modification over the embodiment of Fig. 7, the device 2 of Fig. 8 comprises a bacterial culture medium 24.

As a modification over the embodiment of Fig. 7, the device 2 of Fig. 8 comprises an antibiotic drug 26.

As a modification over the embodiment of Fig. 7, the device 2 of Fig. 8 comprises a plurality of additional inlets 14.

As a modification over the embodiment of Fig. 7, the device 2 of Fig. 8 comprises a plurality of fluid reservoirs 16, each associated with one of the additional inlets 14.

As a modification over the embodiment of Fig. 7, the device 2 of Fig. 8 comprises a plurality of fluid-introduction devices 18.

As compared to the foregoing embodiments, the embodiment of Fig. 8 is thus formed with multiple modifications. According to different embodiments (not shown), the device 2 according to the embodiment of Fig. 1 and Fig. 2, of Fig. 3a, Fig. 3b, of Fig. 4, Fig. 5, Fig. 6, Fig.7 or of any of the other embodiments described in this disclosure is formed with only one or any combination of the modifications described in the context of Fig. 8.

The bacterial culture medium 24 is arranged above the second position and is adapted to support bacterial growth. In the depicted embodiment, it is in the form of a paper strip (more specifically, a strip of blotting paper), impregnated with a material for cultivation of bacteria.

The antibiotic drug 26 is arranged above the second position and is in liquid communication with the bacterial-sensitive indicator 8 arranged above it, as well as with the bacterial culture medium 24 arranged below it. In the depicted embodiment, the antibiotic drug 26 is in the form of a paper strip (more specifically, a strip of blotting paper), impregnated with an antibiotic material.

More specifically, the antibiotic drug 26 is arranged over only a section of the culture medium 24. In other words, a first section of the culture medium 24 is overlain by the antibiotic drug 26. A second section of the culture medium 24 is not overlain by the antibiotic drug 26. In the depicted embodiment, the first and second sections of the culture medium 24 are shown as separate strips of paper, each forming one of said sections. In alternative embodiments (not shown), the culture medium 24 is provided as a single, continuous strip of paper, part thereof overlain by the antibiotic drug 26, and part thereof not overlain by the antibiotic drug 26.

In the depicted embodiment, the device 2 comprises a plurality of additional inlets 14, comprising an additional inlet 14 for each of the first and second section of the culture medium 24. Thus, the additional fluid 40 can be provided separately to the first and second section of the culture medium 24. In alternative embodiments (not shown), however, a single additional inlet 14 for both the first section and the second section of the culture medium 24 is provided.

Optionally, a fluid reservoir 16 is provided for the additional inlet 14, and, further optionally, a fluid-introduction device 18 is provided for the additional inlet 14. According to alternative embodiments (not shown), the fluid reservoir 16 and the fluid-introduction device 18 are provided separately.

Providing the antibiotic drug 26 above a (first) section or some (first) sections of the culture medium 24 allows for the assessment of bacterial susceptibility to the respective antibiotic drug 26, using the (second) section(s) of the culture medium 24 not overlain by the antibiotic drug 26 as a reference.

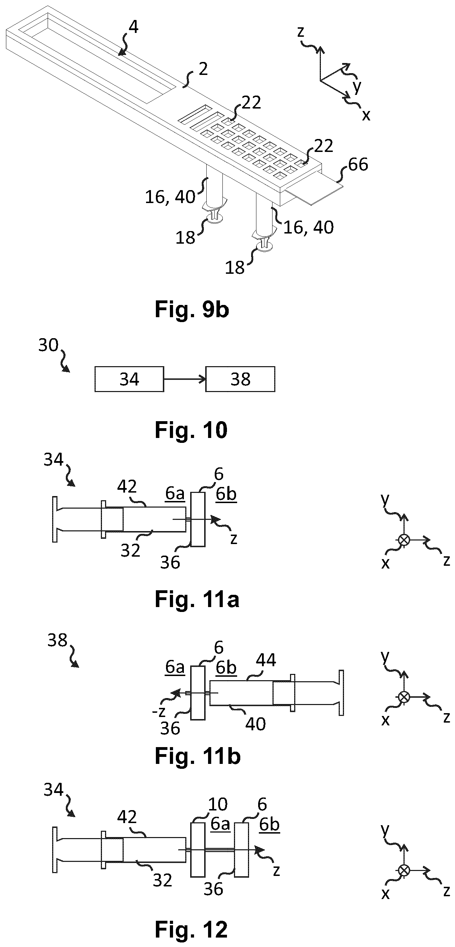

Fig. 9a and Fig. 9b illustrate a device 2 according to a further embodiment. The embodiment of Fig. 9a, Fig. 9b is similar to the embodiment of Fig. 8. Similar elements are indicated with identical reference numerals. To avoid repetition, for a detailed description of these elements, reference is made to the corresponding description above in the context of the foregoing embodiments.

Fig. 9a gives a cross-sectional view of the device 2, and Fig. 9b shows a perspective view thereof.

As illustrated in Fig. 9a and Fig. 9b, the culture media 24 (or, alternatively, sections of a culture medium 24) are preferably arranged in a line or in a two-dimensional array.

An observation window 22 is associated with each of the culture media 24 (or, alternatively, sections of a culture medium 24).

Some of the culture media 24 (or, alternatively, of the sections of a culture medium 24) are overlain by an antibiotic drug 26, whereas others are not.

Fig. 10 illustrates a method 30 for extracting bacteria 36 contained in urine 32. The method 30 comprises two steps: flowing 34 the urine 32 through a filter 6 and flowing 38 an additional fluid 40 through the filter 6. The first step involves flowing 34 the urine 32 through the filter 6 along a first flow direction z from a first side 6a to a second side 6b, retaining the bacteria 36 on the first side 6a. The second step involves flowing 38 the additional fluid 40 through the filter 6 from the second side 6b to the first side 6a, inoculating the bacteria 36 in the additional fluid 40.

Fig. 11a, Fig. 11b illustrate respective method steps 34, 38 in further detail, according to some embodiments.

Fig. 11a schematically illustrates the first step 34 of the method 30, where urine 32 is driven through the filter 6 using a first syringe 42. The filter 6 retains the bacteria 36 on the first side 6a, while the urine 32 passes through to the second side 6b. This step is particularly useful in concentrating bacteria 36 from the urine 32, facilitating subsequent detection and analysis. Undersized contaminants pass through the filter and are thus removed from the bacteria 36.

Fig. 11b illustrates the second step 38 of the method 30, where an additional fluid 40 is driven through the filter 6 using a second syringe 44. The additional fluid 40 flows from the second side 6b to the first side 6a, inoculating the bacteria 36 in the additional fluid 40. This step transfers the bacteria 36 into the additional fluid 40, which is adapted to provide a medium suitable for detection or further analysis.

Fig. 12 depicts an alternative embodiment of the first step 34, where an additional filter 10 is arranged on the first side 6a of the filter 6. The additional filter 10 permits the bacteria 36 to pass through while filtering out larger particles from the urine 32. This additional filtering step enhances the purity of the bacterial sample retained on the filter 6.

In conventional techniques for diagnostics using urine, such as a urine culture, a filtering step is not applied. As laid out above, the filtering is particularly useful in the context of "paper-based" diagnostics, wherein the bacterial-sensitive indicator or the bacterial culture medium is provided in the form of a paper impregnated therewith. The filtering reduces colored and/or strongly absorbing contaminants, significantly improving sensitivity.

This has been proven by the inventors in a clinical study. In the study, the sensitivity of a paper-based test to Escherichia coli bacteria was determined. For this purpose, a paper strip impregnated with a bacterial-sensitive indicator 8 was exposed to a filtered sample or to a reference sample. Different bacterial-sensitive indicator 8 were applied: resazurin, MTT (n = 27), and EI (n = 25).

To obtain the filtered sample, the bacteria were retained in a filter (pore size: 0.45 µm) and back washed as described above in the context of the method 30. Untreated urine 32 was used as the reference sample.

Prior to applying the filtered and reference samples to the paper strip, a bacterial cultivation was performed on the respective samples for 4 h, 8 h, or 12 h.

Sensitivities are given in the following, wherein the bacterial cultivation was performed on the samples for 4 h:

| Resazurin | | MTT | | EI | |
|---|---|---|---|---|---|
| Filtered | Reference | Filtered | Reference | Filtered | Reference |
| 93.8 | 50 | 87.5 | 50 | 80 | 50 |

Sensitivites are given in the following, wherein the bacterial cultivation was performed on the samples for 8 h:

| Resazurin | | MTT | | EI | |
|---|---|---|---|---|---|
| Filtered | Reference | Filtered | Reference | Filtered | Reference |
| 100 | 50 | 100 | 50 | 93.3 | 66.7 |

Sensitivites are given in the following, wherein the bacterial cultivation was performed on the samples for 12 h:

| Resazurin | | MTT | | EI | |
|---|---|---|---|---|---|
| Filtered | Reference | Filtered | Reference | Filtered | Reference |
| 93.8 | 50 | 93.8 | 50 | 80 | 100 |

As can be seen from these results, the filtering yields a surprisingly large improvement in sensitivity, in particular for cell cultivation times below 12 hours, and in particular, for cell cultivation times of 8 h or less.

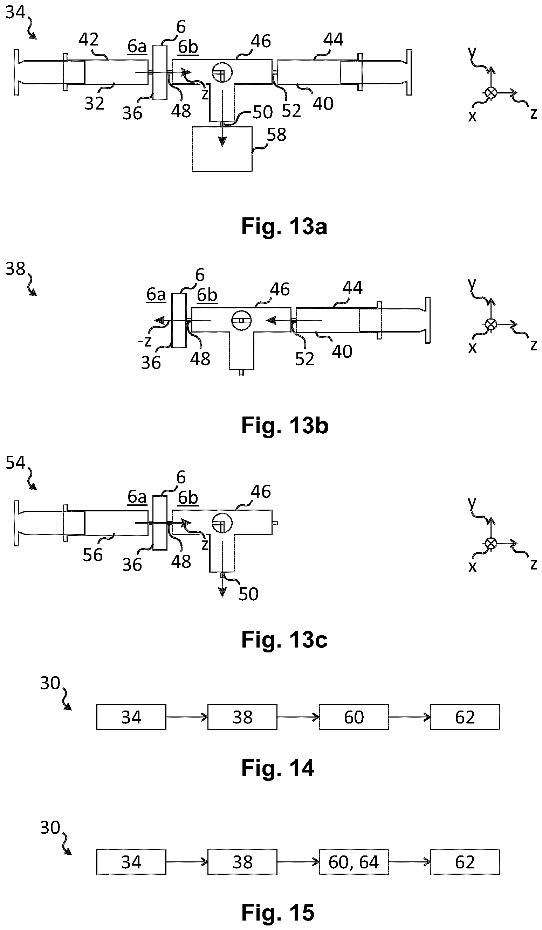

Fig. 13a depicts an alternative embodiment of the first step 34.

According to the embodiment of Fig. 13a, an at-least-three-way valve 46 with a collection reservoir 58 is arranged downstream from the filter 6.

The at-least-three-way valve 46 has a first port 48, a second port 50, and a third port 52.

The collection reservoir 58 is arranged on the second port 50.

At step 34, the at-least-three-way valve 46 is set such that it fluid-connects the first and second ports 48, 50 thereof connected to the filter 6 and to the collection reservoir 58, respectively.

The urine 32 is driven through the filter 6 from the first side 6a to the second side 6b using the first syringe 42, and to the collection reservoir 58.

In more detail, the first syringe 42 is used to drive the urine 32 through the filter 6. The collection reservoir 58 is used to collect the urine 32 after it has passed through the filter 6.

The reservoir 58 helps ensure that the urine 32 is properly contained and can be easily disposed of or analyzed further.

Fig. 13b depicts an alternative embodiment of the second method step 38.

The elements depicted in 13b are similar to the elements depicted in Fig. 13a. For the sake of clarity, corresponding elements are indicated with identical reference numerals. To avoid repetition, for a detailed description of these elements, reference is made to the corresponding description above.

At step 38, the at-least-three-way valve 46 is set such that it fluid-connects the first and third ports 48, 52 thereof connected to the filter 6 and to a second syringe 44, respectively.

The additional fluid 40 is driven through the filter 6 from the second side 6b to the first side 6a using the second syringe 44.

Fig. 13c depicts an optional, additional method step 54, which, according to various embodiments, is applied between process steps 34 and 38.

This additional method step 54 involves flowing a purge liquid 56 through the filter 6 along the first flow direction z. The at-least-three-way valve 46 is adjusted to direct the flow of the purge liquid 56 from the first port 48 to the second port 50.

A respective additional process step 54 helps to ensure that the urine 32 is cleaned from undersized contaminants, which are further flown away from the side 6a through the filter 6 using the purge liquid 56.

The foregoing embodiments have described the method 30 with reference to exemplary embodiments wherein the urine 32, at step 34, is flown through a filter 6 (and, optionally an additional filter 10) on a port 48 of an at-least-three-way valve 46, and wherein the additional fluid 40 is thereafter flown through the filter 6 on the port 48 of the at-least-three-way valve 46. According to alternative embodiments, the method 30 is performed using the filter 6 (optionally, the additional filter 10) of the device 2 described above. In other words, the method 30 according to some embodiments is performed using the device 2 described above. Any of the aforementioned embodiments achieves the technical effect of purifying the urine from contaminants using a backwashing technique, with the advantages described above.

Fig. 14 schematically illustrates a method 30 for extracting bacteria 36 from urine 32 according to a further embodiment. The method 30 comprises flowing 34 the urine 32 through the filter 6, flowing 38 an additional fluid 40 through the filter 6, performing 60 a bacterial cultivation on the bacteria 36, and applying 62 a bacterial-sensitive indicator 8 to the bacteria 36.

Fig. 15 illustrates another alternative embodiment of the method 30, similar to the embodiment of Fig. 14. According to the embodiment of Fig. 14, the method 30 includes an additional step of applying 64 an antibiotic drug 26 to the bacteria 36 during and/or after performing 60 the bacterial cultivation, and during and/or before applying 62 the bacterial-sensitive indicator 8.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A device (2) for detecting bacteria (36) contained in a fluid, the device (2) comprising:
an inlet (4) arranged on the device (2), the inlet (4) adapted to permit the fluid to enter the device (2) therethrough;
a filter (6) adapted to retain the bacteria (36) contained in the fluid; and
a bacterial-sensitive indicator (8) adapted to indicate a presence of the bacteria (36), the bacterial-sensitive indicator (8) arranged at a position horizontally offset from a position of the inlet (4);
wherein the filter (6) is arranged moveably between a first position below the inlet (4) and a second position below the bacterial-sensitive indicator (8).

2. The device (2) according to claim 1, further comprising an additional filter (10) adapted to permit the bacteria (36) contained in a fluid to pass through the additional filter (10) when the fluid containing the bacteria (36) passes through the additional filter (10),
wherein the additional filter (10) is arranged between the first position and the inlet (4),
in particular, wherein the additional filter (10) has pore sizes exceeding 1 µm or exceeding 2 µm or exceeding 3 µm.

3. The device (2) according to any of the preceding claims, further comprising a fluid absorber (12) arranged below the first position, the fluid absorber (12) adapted to provide, when the fluid enters the device (2) through the inlet (4), a capillary suction force acting on the fluid, said capillary suction force being directed from the inlet (4) towards the filter (6) and the fluid absorber (12);
in particular, wherein the fluid absorber (12) comprises or is composed of cotton.

4. The device (2) according to any of the preceding claims, further comprising, below the second position, an additional inlet (14) for introducing, when the filter (6) is at the second position, an additional fluid (40) through the additional inlet (14) and through the filter (6) towards the bacterial-sensitive indicator (8);
wherein, optionally:
the device (2) further comprises a support (28) arranged horizontally adjacent to the fluid absorber (12), the support (28) comprising an upper surface adapted to support the filter (6) at the second position; and/or
the device (2) further comprises a fluid reservoir (16) comprising the additional fluid (40), and a fluid-introduction device (18) adapted to drive the additional fluid (40) from the fluid reservoir (16) through the additional inlet (14); wherein, further optionally, the device (2) further comprises a syringe comprising the fluid reservoir (16) and providing the fluid-introduction device (18).

5. The device (2) according to claim 2, further comprising a separation layer (20) arranged between the first position and the additional filter (10);
wherein, optionally, the separation layer (20) is adapted to provide a sliding layer that reduces sticking of the filter (6) to the additional filter (10) when the filter (6) moves from the first position to the second position.

6. The device (2) according to any of the preceding claims,
wherein the device (2) further comprises an observation window (22) arranged on the device (2) such that the bacterial-sensitive indicator (8) is visible from outside of the device (2) through the observation window (22), in particular, wherein the observation window (22) is arranged above the bacterial-sensitive indicator (8); and/or
wherein the bacterial-sensitive indicator (8) comprises a chromogenic substance and/or a fluorogenic substance; and/or
wherein the bacterial-sensitive indicator (8) comprises a paper, said paper comprising the chromogenic substance and/or the fluorogenic substance; and/or
wherein the bacterial-sensitive indicator (8) comprises or is a redox indicator and/or an enzymatic indicator; in particular, wherein the bacterial-sensitive indicator (8) comprises a paper, said paper comprising the redox indicator and/or the enzymatic indicator.

7. The device (2) according to any of the preceding claims,
wherein the device (2) further comprises a bacterial culture medium (24) adapted to support bacterial growth, said bacterial culture medium (24) being arranged above the second position and in liquid communication with the bacterial-sensitive indicator (8); in particular, wherein the device (2) further comprises a second paper impregnated with the bacterial culture medium (24), the second paper being arranged above the second position and in liquid communication with the bacterial-sensitive indicator (8); and/or
wherein the device (2) further comprises an antibiotic drug (26), said antibiotic drug (26) being arranged above the second position and in liquid communication with the bacterial-sensitive indicator (8); in particular, wherein the device (2) further comprises a third paper impregnated with the antibiotic drug (26), the third paper being arranged above the second position and in liquid communication with the bacterial-sensitive indicator (8); and/or
wherein the filter (6) has pore sizes smaller than 1 µm or smaller than 0.8 µm; and/or
wherein the device (2) further comprises a sliding member (66) slidably mounted to the device, wherein the filter (6) is connected to the sliding member (66) such that the filter (6) moves together with the sliding member (66), and wherein the sliding member (66) and the filter (6) connected thereto are adapted such that the filter (6) moves between the first position and the second position as it moves together with the sliding member (66), wherein optionally:
said sliding member (66) is slidable along the horizontal direction; and/or the sliding member (66) is arranged partially inside of the device and partially outside of the device, in particular, when the filter (6) is at the second position or when the filter (6) is at the first position and when the filter (6) is at the second position; and/or
wherein the bacterial-sensitive indicator (8) is arranged inside the device (2) and/or wherein the first position and/or the second position are/is inside the device (2); and/or
wherein the bacteria (36) are Escherichia coli bacteria; and/or wherein the fluid is urine (32).

8. A method (30) for extracting bacteria (36) contained in urine (32), the method (30) comprising:
flowing (34) the urine (32) through a filter (6) along a first flow direction (z) from a first side (6a) of the filter (6) to a second side (6b) of the filter (6) opposite to the first side (6a), the filter (6) retaining the bacteria (36) contained in the urine (32) on the first side (6a) of the filter (6);
thereafter, flowing (38) an additional fluid (40) through the filter (6) from the second side (6b) of the filter (6) to the first side (6a) of the filter (6), thus inoculating the bacteria (36) in the additional fluid (40) flown to said first side (6a).

9. The method (30) according to claim 8, wherein, in the process step of flowing (34) the urine (32) through the filter (6),
an additional filter (10) adapted to permit the bacteria (36) contained in the urine (32) to pass therethrough is arranged on the first side (6a) of the filter (6), and
the urine (32) is flown through the additional filter (10) and through the filter (6).

10. The method (30) according to claim 9,
wherein, in the process step of flowing (38) the additional fluid (40) through the filter (6) from the second side (6b) of the filter (6) to the first side (6a) of the filter (6), the additional fluid (40) is not flown through the additional filter (10); and/or
wherein the method (30) further comprises, in between the process steps of flowing (34) the urine (32) through the filter (6) and flowing (38) the additional fluid (40) through the filter (6), removing the additional filter (10) from the first side (6a) of the filter (6).

11. The method (30) according to any of claims 8 to 10,
wherein the process step of flowing (34) the urine (32) through the filter (6) comprises flowing (34) the urine (32) through an at-least-three-way valve (46) downstream from the filter (6) from a first port (48) of the at-least-three-way valve (46) to a second port (50) of the at-least-three-way valve (46); and
wherein the process step of flowing (38) the additional fluid (40) through the filter (6) from the second side (6b) of the filter (6) to the first side (6a) of the filter (6) comprises flowing (38) the additional fluid (40) through the at-least-three-way valve (46) from a third port (52) of the at-least-three-way valve (46) to the first port (48) of the at-least-three-way valve (46), the at-least-three-way valve (46) being arranged upstream from the filter (6) in the respective process step.
wherein, optionally, the method (30) further comprises, between the process steps of flowing (34) the urine (32) through the filter (6) and flowing (38) the additional fluid (40) through the filter (6), flowing (54) a purge liquid (56) through the filter (6) along the first flow direction (z); wherein, further optionally, a volume of the purge liquid (56) flown through the filter (6) exceeds a volume of the urine (32) flown through the filter (6).

12. The method (30) according to claim 11, wherein a collection reservoir (58) is arranged on the second port (50) of the at-least-three-way valve (46), and wherein the collection reservoir (58) receives the urine (32) flown through the at-least-three-way valve (46) from the first port (48) of the at-least-three-way valve (46) to the second port (50) of the at-least-three-way valve (46);
wherein, optionally, the collection (58) reservoir comprises or is a collection bag (58).

13. The method (30) according to any of claims 8 to 12,
which further comprises, after inoculating the bacteria (36), performing (60) a bacterial cultivation on the bacteria (36), wherein, optionally, the bacterial cultivation is performed using a bacterial culture medium (24) impregnated in a second paper, wherein, further optionally, the method (30) is performed using the filter (6) and the second paper of the device (2) according to claim 7; and/or
which further comprises, after inoculating the bacteria (36), applying (62) a bacterial-sensitive indicator (8) to the bacteria (36) to identify the bacteria (36), wherein, optionally, the bacterial-sensitive indicator (8) is impregnated in a paper, wherein, further optionally, the method is performed using the filter (6) and the paper of the device (2) according to claim 6; and/or
which, after inoculating the bacteria (36), further comprises applying (64) an antibiotic drug (26) to the bacteria (36), wherein, optionally, the antibiotic drug (26) is impregnated in a third paper, wherein, further optionally, the method is performed using the filter (6) and the third paper of the device (2) according to claim 7; and/or
which further comprises, after inoculating the bacteria (36):
performing (60) a bacterial cultivation on the bacteria (36), and applying (62) a bacterial-sensitive indicator (8) to the bacteria (36) to identify the bacteria (36); or
performing (60) a bacterial cultivation on the bacteria (36) and applying (64) an antibiotic drug (26) to the bacteria (36), and applying a bacterial-sensitive indicator (8) to the bacteria (36) to identify the bacteria (36), wherein, optionally, the antibiotic drug (26) is impregnated in a third paper, wherein, further optionally, the method is performed using the filter (6) and the third paper of the device (2) according to claim 7;
wherein, optionally:
the bacterial-sensitive indicator (8) is impregnated in a paper, wherein, further optionally, the method is performed using the filter (6) and the paper of the device (2) according to claim 6; and/or
the bacterial cultivation is performed using a bacterial culture medium (24) impregnated in a second paper, wherein, further optionally, the method (30) is performed using the filter (6) and the second paper of the device (2) according to claim 7; and/or
wherein the process step of flowing (34) the urine (32) through the filter (6) comprises driving the urine (32) through the filter (6) using a first syringe (42); and/or
wherein the process step of flowing (38) the additional fluid (40) through the filter (6) comprises driving the additional fluid (40) through the filter (6) using a second syringe (44);

14. The method (30) according to claim 8, which is performed using the filter (6) of the device (2) according to any of claims 1 to 7.

15. The method (30) according to claim 9, which is performed using the filter (6) of the device (2) according to claim 2 and using the additional filter (10) of the device (2) according to claim 2.
